# EUROPÄISCHE PATENTSCHRIFT

(11) **EP 1 948 260 B1**
(45) Veröffentlichungstag und Bekanntmachung des Hinweises auf die Patenterteilung: **20.01.2010**
(21) Anmeldenummer: 06818565.1
(22) Anmeldetag: 16.11.2006
(51) Int. Cl.: A61L 27/36, A61L 27/38, A61L 27/48, A61L 27/56

(54) **VERBUNDMATERIAL, INSBESONDERE FÜR DIE MEDIZINISCHE ANWENDUNG, UND VERFAHREN ZU DESSEN HERSTELLUNG**
COMPOSITE MATERIAL, ESPECIALLY FOR MEDICAL USE, AND METHOD FOR PRODUCING THE SAME
MATERIAU COMPOSITE UTILISE EN PARTICULIER EN MEDECINE ET PROCEDE DE PRODUCTION ASSOCIE

(30) Priorität: 17.11.2005 DE 102005054940
(43) Veröffentlichungstag der Anmeldung: 30.07.2008
(73) Patentinhaber: GELITA AG, 69412 Eberbach (DE); Tetec-Tissue Engineering Technologies Aktiengesellschaft, 72770 Reutlingen (DE)
(72) Erfinder: AHLERS, Michael, 69412 Eberbach (DE); BADZIONG, Werner, 69412 Eberbach (DE); GAISSMAIER, Christoph, 72127 Kusterdingen (DE); FRITZ, Jürgen, 72144 Dusslingen (DE)
(74) Vertreter: Wössner, Gottfried
(86) Internationale Anmeldenummer: PCT/EP2006/010972
(87) Internationale Veröffentlichungsnummer: WO 2007/057175

(56) Entgegenhaltungen:
- EP-A- 0 237 037
- EP-A- 1 031 356
- EP-A- 1 201 202
- WO-A-2005/111121
- WO-A-2006/045330
- US-A- 5 201 745
- ULUBAYRAM K ET AL: "EGF containing gelatin-based wound dressings" BIOMATERIALS, ELSEVIER SCIENCE PUBLISHERS BV., BARKING, GB, Bd. 22, Nr. 11, 1. Juni 2001 (2001-06-01), Seiten 1345-1356, XP004238847 ISSN: 0142-9612

## Beschreibung

Die vorliegende Erfindung betrifft ein bioverträgliches, resorbierbares Verbundmaterial, das insbesondere als Matrixmaterial im human- und veterinärmedizinischen Bereich zur Anwendung kommt. Solche Materialien können sowohl zellfrei als auch mit Zellen besiedelt zum Einsatz kommen.

Die Erfindung betrifft ferner ein Verfahren zur Herstellung eines derartigen Verbundmaterials.

Schließlich betrifft die Erfindung Implantate, insbesondere Zell- und Gewebeimplantate, die unter Verwendung des Verbundmaterials hergestellt sind, und deren Anwendung zur Behandlung des menschlichen oder tierischen Körpers.

Bei Schädigungen mancher menschlicher oder tierischer Gewebe, die sowohl krankheits- als auch verletzungsbedingt sein können, kommen zur Unterstützung des Heilungsprozesses resorbierbare Implantate zur Anwendung. Diese fördern die Regeneration des betroffenen Gewebes, indem sie eine mechanische Schutzfunktion für das sich neu bildende Gewebe ausüben und/oder eine das Zellwachstum fördernde Matrix bilden.

Ein bedeutendes Anwendungsgebiet für derartige Implantate stellt das Knorpelgewebe dar. Dieses besteht aus Chondrozyten (Knorpelzellen) und der von diesen Zellen synthetisierten extrazellulären Matrix, welche hauptsächlich aus Kollagen und Proteoglykanen aufgebaut ist. Da der Knorpel nicht durchblutet ist, überwiegend durch Diffusion ernährt wird und nach dem Schließen der Wachstumsfugen keinen direkten Zugang zu regenerativen Zellpopulationen aufweist, verfügt er nur über eine limitierte intrinsische Regenerationsfähigkeit. Die selbstständige Ausheilung eines Knorpelschadens ist daher vor allem beim Erwachsenen nur sehr eingeschränkt möglich und wird selten beobachtet. Knorpeldefekte können durch Verletzungen oder degenerative Einflüsse entstehen und führen ohne biologisch rekonstruktive Intervention häufig zu einem weiteren Fortschreiten der Knorpelschädigung bis hin zur destruktiven Osteoarthrose.

Bei einer besonderen Behandlungsform der oben beschriebenen Knorpelschäden werden Chondrozyten zunächst *in vitro* auf einem resorbierbaren Implantat mit Hilfe einer Nährlösung kultiviert. Anschließend wird ein so hergestelltes Zell-Träger-Konstrukt in die Stelle des fehlenden oder geschädigten Knorpels eingesetzt. Die angezüchteten Chondrozyten werden zuvor dem Patienten selbst entnommen, so dass man bei diesem Verfahren auch von der autologen Knorpelzelltransplantation spricht. Nach der Implantation produzieren die Zellen neue extrazelluläre Matrix und führen so zur Defektausheilung. Das Trägermaterial wird im Verlauf der Regeneration abgebaut (resorbiert). Außer der Verwendung von autologen Chondrozyten ist auch die Implantation von allogenen Chondrozyten oder die Verwendung von chondrogen *in vitro* vordifferenzierten Stammzellen (autolog oder allogen) denkbar und wird derzeit in präklinischen und tierexperimentellen Untersuchungen auf die klinische Anwendbarkeit beim Menschen überprüft.

Neben der autologen Chondrozytentransplantation bilden knochenmarkstimulierende Verfahren, wie Mikrofrakturierung oder Anbohrung, eine weitere klinisch etablierte Therapie mit biologisch rekonstruktiver Zielsetzung bei Gelenkknorpelschäden. Bei diesen Methoden wird nach vorherigem Debridement mit kleinen Ahlen oder Bohrern die subchondrale Knochenplatte perforiert, wodurch es zu einer Einblutung in den Defektbereich kommt unter Bildung eines Blutkoagels. Im weiteren Verlauf entsteht aus dem Blutkoagel (sog. Superclot) ein Faserknorpel, der in vielen Fällen zu einer Defektauffüllung und Beschwerdelinderung führt. Durch den Einsatz geeigneter und biokompatibler Matrizes können die Ergebnisse dieser Methode weiter verbessert werden. Das verwendete Biomaterial fixiert den entstandenen Superclot im Defektbereich, schützt ihn vor Abscherung und dient den über den Blutweg eingewanderten Zellen als primäre Matrix für die Defektausheilung.

Als ein weiterer Anwendungsbereich von Biomaterialien kommt die Behandlung von Rupturen der Rotatorenmanschette der Schulter oder die Behandlung einer Teildegeneration der Rotatorenmanschette in Betracht. Zellfreie Biomaterialien werden für diese Indikation zwar bereits verwendet, besitzen jedoch den Nachteil, dass sie ohne vorherige Zellbesiedelung nicht aktiv zur Regeneration beitragen können. Für die Vitalisierung des Biomaterials kann Sehnengewebe bioptisch gewonnen werden. Anschließend werden die Zellen *in vitro* isoliert, angezüchtet, auf ein geeignetes Biomaterial ausgesät und mit diesem in den Defektbereich implantiert.

Eine weitere Anwendung zellbesiedelter Biomaterialien ist die Knochenregeneration, z.B. im Bereich des Kiefers zur Sinusaugmentation, mit vorkultivierten autologen Periostzellen oder mesenchymalen Stammzellen, die auf die Matrix ausgesät werden.

Neben den bisher genannten Indikationen können Biomaterialien in Verbindung mit oder ohne vorherige Zellbesiedelung auch für die Behandlung und Ausheilung von chronischen Wunden, Hautverletzungen oder -verbrennungen verwendet werden.

Für die oben beschriebenen Indikationen und Methoden geeignete Biomaterialien müssen für ihre Anwendbarkeit beim Menschen oder Tier jedoch eine Reihe von Voraussetzungen erfüllen. Von großer Bedeutung ist dabei zunächst eine vollständige Bioverträglichkeit des Materials, d.h. es sollten keine Entzündungs-, Abstoßungs- oder andere Immunreaktionen nach Implantation auftreten. Außerdem sollte das Biomaterial keine negativen Effekte auf das Wachstum oder den Stoffwechsel der transplantierten oder einwandernden Zellen ausüben und nach einer,bestimmten Zeit im Körper vollständig resorbiert werden. Darüber hinaus sollte das Material eine solche Struktur aufweisen, dass es möglichst gleichmäßig von Zellen besiedelt und durchdrungen werden kann.

Gleichzeitig sind auch an die mechanischen Eigenschaften der verwendeten Materialien hohe Anforderungen zu stellen. Eine sichere Handhabung des Materials beim Implantieren, ohne dass es zu Beschädigungen kommt, ist nur bei einer hohen mechanische Festigkeit zu gewährleisten. Insbesondere muss diese Festigkeit auch bei Gewebeimplantaten, die bereits mit Zellen besiedelt sind, gegeben sein.

Neuere Entwicklungen zeigen, dass diese Anforderungen am ehesten mit mehrlagigen Verbundmaterialien zu erfüllen sind. Beispielsweise wird in der WO 99/19005 eine mehrlagige Membran beschrieben, die eine Matrixschicht aus Kollagen-Typ-II mit einer schwammartigen Textur sowie mindestens eine Barriereschicht mit einer geschlossenen, relativ undurchlässigen Textur umfasst.

In dem Artikel "EGF containing gelatin-based wound dressings" von K. Ulubayram et al. (Biomaterials (2001) 22, 1345-1356) wird ein zweilagiges Verbundmaterial beschrieben, welches eine poröse innere Lage aus vernetzter Gelatine umfasst, die einen Wachstumsfaktor zur Unterstützung der Wundheilung enthält, sowie eine äußere Lage aus einer elastomeren Polyurethanmembran, die dem Schutz der Wunde dient.

Die internationale Patentanmeldung WO 2006/045330 A1 (Artikel 54(3) EPÜ) offenbart ein Implantat zur Behandlung von Knorpeldefekten, umfassend eine erste Lage und eine zweite Lage, wobei die erste Lage eine membranartige Struktur umfasst und die zweite Lage eine schwammartige Struktur. Das Material der zweiten Lage kann z.B. Kollagen, Hyaluronsäure, Alginat, Chitosan oder Gelatine umfassen.

Die internationale Patentanmeldung WO 2005/111121 A2 (Artikel 54(3) EPÜ) offenbart ein Verfahren zur Herstellung von Formkörpern auf Basis von vernetzter Gelatine, welches auf einer zweistufigen Vernetzung der Gelatine basiert. Bei den auf diese Weise hergestellten Formkörpern kann es sich unter anderem um Folien, Schwämme oder Verbundmaterialien aus Folien und Schwämmen handeln. In der EP 1 263 485 B1 wird ein biokompatibles Mehrlagenmaterial offenbart, welches eine erste und eine zweite Schicht mit Matrizen von biokompatiblem Kollagen umfasst.

Kollagen ist ein natürliches Material mit einer relativ hohen Festigkeit, so dass auf seiner Grundlage Implantate mit guten mechanischen Eigenschaften und einer guten Handhabbarkeit hergestellt werden können. Andererseits hat die Verwendung von Kollagen als Matrix für die Zellen jedoch den Nachteil, dass aufgrund der nicht exakt reproduzierbaren Zusammensetzung und Reinheit von Kollagen Probleme bei der Bioverträglichkeit auftreten können. Ferner kann die Resorptionszeit von Kollagen enthaltenden Materialien kaum beeinflusst werden, was im Hinblick auf die unterschiedlichen Einsatzgebiete wünschenswert wäre.

Die Aufgabe der vorliegenden Erfindung besteht darin, ein Verbundmaterial zur Verfügung zu stellen, bei dem diese Nachteile weitgehend vermieden werden, und das gegenüber den bekannten Materialien verbesserte Eigenschaften aufweist.

Diese Aufgabe wird bei dem Verbundmaterial der eingangs genannten Art erfindungsgemäß dadurch gelöst, dass das Verbundmaterial folgende zwei Lagen umfasst:
- eine erste selbsttragende Lage, die ein unter physiologischen Bedingungen unlösliches, resorbierbares, nicht vergelendes erstes Material umfasst; und
- eine zweite Lage, hergestellt auf der Basis eines vernetzten, Gelatine enthaltenden zweiten Materials, wobei die zweite Lage eine überwiegend offenporige Struktur aufweist.

Bei dem erfindungsgemäßen Verbundmaterial gewährleistet die erste Lage die erforderliche mechanische Festigkeit, während die zweite Lage eine Matrix für das Wachstum von Zellen bildet.

Das erste Material ist unter physiologischen Bedingungen unlöslich und nicht vergelend. Dies bedeutet im Sinne der vorliegenden Erfindung, dass das Material in einer wässrigen Lösung unter den im Körper vorherrschenden Bedingungen (insbesondere Temperatur, pH-Wert und Ionenstärke) nicht physikalisch aufgelöst wird und auch nicht durch Aufnahme von Wasser in ein Gel oder einen gelartigen Zustand übergeht. Eine Gelbildung liegt in diesem Sinne dann vor, wenn das erste Material dadurch seine ursprüngliche Festigkeit und Formhaltigkeit in einem wesentlichen Umfang verliert. Dies schließt jedoch nicht aus, dass das Material bestimmte Mengen an Wasser aufnimmt und dabei gegebenenfalls auch quillt, solange dies zu keiner wesentlichen Beeinträchtigung der mechanischen Festigkeit führt.

Durch die aufgeführten Eigenschaften bleibt das erste Material auch in einem hydratisierten Zustand mechanisch fest und formstabil, wodurch der ersten Lage ihre selbsttragende Funktion verliehen wird. Dies bedeutet nicht nur, dass die erste Lage ohne einen zusätzlichen Träger handhabbar ist, sondern dass sie ihrerseits in der Lage ist, als Träger für die zweite Lage zu dienen.

Gleichzeitig ist das erste Material resorbierbar, d.h. es wird nach einer bestimmten Zeit im Körper durch Hydrolyse abgebaut. An diesem hydrolytischen Abbau können auch Enzyme beteiligt sein. Bevor die Resorption im Körper erfolgt, also insbesondere während einer Kultivierung von Zellen auf dem Verbundmaterial *in vitro* und beim Implantieren des Verbundmaterials, ist die Trägerfunktion der ersten Lage weitgehend unbeeinträchtigt, wodurch dem Verbundmaterial insgesamt die erforderliche mechanische Festigkeit verliehen wird.

Durch die erfindungsgemäße Ausführung der ersten Lage wird damit eine sichere und beschädigungsfreie Handhabung des Verbundmaterials gewährleistet. Dies gilt insbesondere auch für den Fall, wenn die zweite Lage bereits vor dem Implantieren mit Zellen besiedelt ist.

Darüber hinaus bietet die erste Lage auch einen mechanischen Schutz für die Zellen, nachdem das Verbundmaterial implantiert worden ist. Dies ist sowohl für die Transplantation *in vitro* vorkultivierter Zellen als auch für eine matrixgekoppelte Mikrofrakturierung bedeutsam. Bei beiden Methoden wird das Biomaterial vorteilhafterweise so eingesetzt wird, dass die erste Lage vom Knochen weg nach außen orientiert ist. Diese schützt dann die anwachsenden Zellen in der zweiten Lage vor Abscherung und regenerationsstörenden Einflüssen aus dem Gelenkbinnenraum, wie z.B. übermäßige mechanische Belastung.

Ein weiterer Vorteil, der auf der hohen Festigkeit der ersten Lage beruht, ist die chirurgische Vernähbarkeit oder auch die übungsstabile subchondrale Verankerung mittels resorbierbarer Anker des erfindungsgemäßen Verbundmaterials. Die erste Lage weist bevorzugt eine solche Reißfestigkeit auf, dass das Verbundmaterial beim Einnähen oder durch transossäre Verankerung mit Hilfe resorbierbarer Minipins nicht ausreißt.

Vorzugsweise weist die erste Lage eine Reißfestigkeit von 20 N/mm² oder mehr auf.

Bei einer bevorzugten Ausführungsform der Erfindung ist das unlösliche, resorbierbare, nicht vergelende erste Material ein Flächenmaterial auf Basis von Kollagen. Hierunter fallen Flächenmaterialien, die im Wesentlichen aus Kollagen gebildet sind, und bei denen es sich bevorzugt um natürliche Membranen tierischen Ursprungs handelt. Tierische Membranen, die fast ausschließlich aus Kollagen bestehen, können gewonnen werden, indem die Membranen von Fremdbestandteilen, die sich nachteilig auf die Bioverträglichkeit auswirken könnten, befreit werden.

Tierische Membranen verfügen in der Regel über hohe Festigkeiten und eignen sich daher besonders gut als erste Lage des erfindungsgemäßen Verbundmaterials. Insbesondere weist Kollagen die geforderten Eigenschaften dahingehend auf, dass es unter physiologischen Bedingungen unlöslich, nicht vergelend und resorbierbar ist.

Als bevorzugtes Flächenmaterial auf Basis von Kollagen wird als erste Lage des Verbundmaterials eine Perikardmembran verwendet. Beim Perikard handelt es sich um die äußere Schicht des Herzbeutels, die eine besonders reißfeste tierische Membran darstellt. Beispielsweise kann die Perikardmembran vom Rind eingesetzt werden.

Die Perikardmembran weist, wie viele andere tierische Membranen, eine raue und eine glatte Seite auf. Bevorzugt werden solche Membranen in dem Verbundmaterial so eingesetzt, dass die raue Seite zu der zweiten Lage hin orientiert ist. Durch die Rauheit der Oberfläche wird die Stabilität der Verbindung zwischen den beiden Lagen erhöht.

Bei einer weiteren bevorzugten Ausführungsform des erfindungsgemäßen Verbundmaterials umfasst das erste Material einen Verstärkungsstoff. Auch mit Hilfe von unlöslichen, resorbierbaren, nicht vergelenden Verstärkungsstoffen kann die Festigkeit der ersten Lage soweit erhöht werden, dass sie die oben beschriebenen vorteilhaften Eigenschaften aufweist.

Bei Verwendung von Verstärkungsstoffen als erstes Material umfasst die erste Lage vorzugsweise eine Matrix, in die der Verstärkungsstoff eingebettet ist.
Die erste Lage ist dann beispielsweise eine verstärkte Folie. Die Matrix muss dabei ebenfalls resorbierbar sein und umfasst bevorzugt Gelatine.

Eine Gelatine umfassende Matrix der ersten Lage, z.B. eine Gelatinefolie, ist bevorzugt auf Basis eines vernetzten, Gelatine enthaltenden Materials hergestellt. Eine Vernetzung ist in der Regel erforderlich, um das Material in eine unlösliche Form zu überführen. Bevorzugte Ausführungsformen der Vernetzung des Gelatine enthaltenden Materials, insbesondere der Gelatine selbst, sind weiter unten im Zusammenhang mit der zweiten Lage des Verbundmaterials ausgeführt.

Die Verstärkungsstoffe zeigen schon bei Anteilen von 5 Gew.%, bezogen auf die Masse der ersten Lage, eine merkliche Verbesserung der mechanischen Eigenschaften derselben.

Oberhalb von 60 Gew.% lässt sich in der Regel keine signifikante Verbesserung mehr erreichen und/oder die gewünschten Resorptionseigenschaften oder auch die notwendige Flexibilität der ersten Lage lassen sich nur noch schwierig darstellen. Die Verstärkungsstoffe lassen sich aus partikulären und molekularen Verstärkungsstoffen sowie Mischungen hiervon auswählen.

Bei den partikulären Verstärkungsstoffen empfiehlt sich insbesondere die Verwendung von Verstärkungsfasern. Die Fasern sind dabei bevorzugt ausgewählt aus Polysaccharid- und Proteinfasern, insbesondere Kollagenfasern, Seide und Baumwollfasern, sowie aus Polylactidfasern und Mischungen hiervon.

Andererseits sind molekulare Verstärkungsstoffe ebenso geeignet, um die mechanischen Eigenschaften und, falls gewünscht, auch die Resorptionsstabilität der ersten Lage zu verbessern.

Bevorzugte molekulare Verstärkungsstoffe sind insbesondere Polylactidpolymere und deren Derivate, Cellulosederivate und Chitosan und dessen Derivate. Auch lassen sich die molekularen Verstärkungsstoffe als Mischungen einsetzen.

Die zweite Lage des erfindungsgemäßen Verbundmaterials ist diejenige Lage, die bei der medizinischen Anwendung unmittelbar mit den Zellen in Kontakt kommt, und die daher in der Lage sein soll, als Substrat für die Besiedlung mit Zellen und als Matrix für deren Wachstum zu fungieren. Aus diesem Grund sind an die Bioverträglichkeit (d.h. Zellverträglichkeit) des zweiten Materials besonders hohe Anforderungen zu stellen. Da die erste Lage bereits die erforderliche mechanische Festigkeit des Verbundmaterials gewährleistet und eine Tragefunktion für die zweite Lage erfüllt, kann bei der Auswahl von Material und Struktur der zweiten Lage ganz auf deren Bioverträglichkeit und biologische Funktionalität abgestellt werden.

Die genannten Anforderungen an die zweite Lage werden durch den erfindungsgemäßen Einsatz von Gelatine in weitem Umfang erfüllt. Gelatine ist, im Gegensatz zu Kollagen, in definierter und reproduzierbarer Zusammensetzung sowie in hoher Reinheit erhältlich. Sie weist eine hervorragende Gewebe- und Zellverträglichkeit auf und ist rückstandslos resorbierbar.

Bevorzugt ist das zweite Material zu überwiegende Anteilen aus Gelatine gebildet, weiter bevorzugt ist es im Wesentlichen vollständig aus Gelatine gebildet.

Um eine optimale Bioverträglichkeit der zweiten Lage des erfindungsgemäßen Verbundmaterials bei der medizinischen Anwendung zu gewährleisten, enthält das zweite Material bevorzugt eine Gelatine mit einem besonders geringen Gehalt an Endotoxinen. Bei Endotoxinen handelt sich um Stoffwechselprodukte oder Bruchstücke von Mikroorganismen, welche in dem tierischen Rohmaterial vorkommen. Der Endotoxingehalt von Gelatine wird in internationalen Einheiten pro Gramm (I.E./g) angegeben und gemäß dem LAL-Test bestimmt, dessen Durchführung in der vierten Ausgabe des Europäischen Arzneibuches (Ph. Eur. 4) beschrieben ist.

Um den Gehalt an Endotoxinen möglichst gering zu halten, ist es vorteilhaft, die Mikroorganismen möglichst frühzeitig im Zuge der Gelatineherstellung abzutöten. Ferner sollten entsprechende Hygienestandards beim Herstellungsprozess eingehalten werden.

Somit kann der Endotoxingehalt von Gelatine durch bestimmte Maßnahmen beim Herstellungsprozess drastisch gesenkt werden. Zu diesen Maßnahmen zählen in erster Linie die Verwendung frischer Rohmaterialien (z.B. Schweineschwarte) unter Vermeidung von Lagerzeiten, die sorgfältige Reinigung der gesamten Produktionsanlage unmittelbar vor Beginn der Gelatineherstellung sowie gegebenenfalls das Auswechseln von Ionenaustauschern und Filtersystemen in der Produktionsanlage.

Die im Rahmen der vorliegenden Erfindung eingesetzte Gelatine weist bevorzugt einen Endotoxingehalt von 1.200 I.E./g oder weniger, noch mehr bevorzugt von 200 I.E/g oder weniger auf. Optimalerweise liegt der Endotoxingehalt bei 50 I.E./g oder weniger, jeweils gemäß dem LAL-Test bestimmt. Im Vergleich hierzu weisen manche handelsübliche Gelatinen Endotoxingehalte von über 20.000 I.E./g auf.

Erfindungsgemäß ist das zweite, Gelatine enthaltende Material vernetzt, wobei vorzugsweise die Gelatine vernetzt ist. Da Gelatine an sich wasserlöslich ist, ist eine Vernetzung in der Regel erforderlich, um ein zu schnelles Auflösen des zweiten Materials zu verhindern, und damit auch für die zweite Lage des Verbundmaterials eine ausreichende Lebensdauer unter physiologischen Bedingungen zu gewährleisten.

Dabei bietet Gelatine den zusätzlichen Vorteil, dass die Resorptionsgeschwindigkeit des vernetzten Materials, bzw. die Zeitdauer bis zur vollständigen Resorption, durch die Wahl des Vernetzungsgrades über einen weiten Bereich eingestellt werden kann.

Das zweite Material ist bevorzugt chemisch vernetzt. Als Vernetzungsmittel können dabei prinzipiell alle Verbindungen eingesetzt werden, die eine chemische Vernetzung der Gelatine bewirken. Bevorzugt sind Aldehyde, Dialdehyde, Isocyanate, Diisocyanate, Carbodiimide und Alklydihalogenide. Besonders bevorzugt ist Formaldehyd, da dieses gleichzeitig einen sterilisierenden Effekt aufweist.

Um dabei die Bioverträglichkeit des zweiten Materials sicherzustellen, ist dieses vorzugsweise im Wesentlichen frei von überschüssigem, d.h. nicht reagiertem Vernetzungsmittel. Bevorzugt liegt dabei der Gehalt an überschüssigem Vernetzungsmittel bei ca. 0,2 Gew.% oder weniger, was insbesondere im. Fall von Formaldehyd einen Grenzwert für die Zulassung als Implantatmaterial derstellt.

Bei einer weiteren Ausführungsform ist das zweite Material enzymatisch vernetzt. Als Vernetzungsmittel wird dabei bevorzugt das Enzym Transglutaminase eingesetzt, welches eine Verknüpfung der Glutamin- und Lysinseitenketten von Proteinen, insbesondere auch von Gelatine, bewirkt.

Die aufgeführten Vernetzungsmittel sind ebenso geeignet zur Vernetzung des Gelatine enthaltenden Materials der ersten Lage, in dem Fall, dass diese eine Gelatine enthaltende Matrix mit einem eingelagerten Verstärkungsstoff umfasst.

Neben der Bioverträglichkeit des verwendeten Materials sollte die zweite Lage des Verbundmaterials auch so beschaffen sein, dass sie eine für die Besiedlung mit Zellen geeignete Struktur aufweist. Erfindungsgemäß wird dies durch die überwiegend offenporige Struktur gewährleistet, die ein Eindringen von Zellen in die Struktur sowie eine möglichst gleichmäßige Verteilung der Zellen über die gesamte Dicke der zweiten Lage ermöglicht.

Die überwiegend offenporige Struktur wird bei einer bevorzugten Ausführungsform der Erfindung dadurch verwirklicht, dass die zweite Lage eine Faserstruktur aufweist. Die Faserstruktur umfasst bevorzugt ein Gewebe, ein Gewirk oder ein Vlies. Faserstrukturen können aus dem Gelatine enthaltenden zweiten Material beispielsweise durch Extrudieren oder Elektrospinnen einer Gelatinelösung hergestellt werden.

Bei einer weiteren bevorzugten Ausführungsform des erfindungsgemäßen Verbundmaterials weist die zweite Lage eine Schwammstruktur auf. Schwammstrukturen können durch Aufschäumen einer Lösung des Gelatine enthaltenden zweiten Materials hergestellt werden, worauf im Zusammenhang mit dem erfindungsgemäßen Herstellungsverfahren noch näher eingegangen wird.

Schwammstrukturen mit überwiegend offenen Poren eignen sich in besonderem Maße für eine Besiedlung mit Zellen. Durch die miteinander verbundenen Hohlräume kann eine sehr gleichmäßige Verteilung der Zellen über das gesamte Volumen erreicht werden. Bei Wachstum der Zellen und Synthese der extrazellulären Matrix bildet sich so eine dreidimensionale Gewebestruktur aus. Dies geht mit einem sukzessiven hydrolytischen Abbau des vernetzten, Gelatine enthaltenden Materials einher, so dass das Volumen der Schwammstruktur nach dem vollständigen Abbau des Materials (bzw. nach dessen Resorption im Körper) weitgehend von dem neu gebildeten Gewebe eingenommen wird.

Der bevorzugte mittlere Porendurchmesser der Schwammstruktur richtet sich in erster Linie nach der Größe der Zellen, mit denen das Verbundmaterial *in vitro* oder *in vivo* besiedelt werden soll. Bei zu kleinen Porendurchmessern können die Zellen nicht in die Struktur eindringen, während zu große Poren einen zu geringen Rückhaltegrad beim Einbringen oder Einwachsen der Zellen zur Folge haben. Vorzugsweise liegt der mittlere Porendurchmesser unterhalb von 500 µm, insbesondere im Bereich von 100 bis 300 µm.

Die Porengröße der Schwammstrukturen ist zu einem Großteil von deren Dichte abhängig. Die Dichte der zweiten Lage des Verbundmaterials, insbesondere im Fall einer Schwammstruktur, liegt bevorzugt im Bereich von 10 bis 100 g/l, weiter bevorzugt 10 bis 50 g/l, am meisten bevorzugt 15 bis 30 g/l. Die Dichte von Schwammstrukturen kann dabei durch die Herstellungsbedingungen, insbesondere durch die Intensität des Aufschäumens, beeinflusst werden.

Bevorzugt ist die zweite Lage des erfindungsgemäßen Verbundmaterials in hydratisiertem Zustand elastisch verformbar, insbesondere im Fall einer Schwammstruktur. Ein hydratisierter Zustand liegt vor, wenn das Verbundmaterial in einer wässrigen Umgebung soviel Wasser aufgenommen hat, dass im Wesentlichen ein Gleichgewichtszustand erreicht ist. Solche Bedingungen liegen sowohl bei einer Kultivierung von Zellen in einem Nährmedium *in vitro* als auch im Körper vor.

Das Ausmaß der elastischen Verformbarkeit lässt sich beispielsweise über das Dekompressionsverhalten definieren. Bevorzugt ist die zweite Lage so ausgebildet, dass sie sich nach einer Volumenkompression durch Beaufschlagung mit einem Druck von 22 N/cm² in hydratisiertem Zustand innerhalb von 10 Minuten zu 90% oder mehr dekomprimiert, was mit Materialien auf der Basis von Kollagen in der Regel nicht erreicht werden kann. Zur Messung des Dekompressionsverhaltens in hydratisiertem Zustand wird das zu prüfende Material bei 37°C in PBS-Puffer (pH 7,2) eingelegt.

Solche elastisch verformbare Strukturen führen zu einer Flexibilität der zweiten Lage des Verbundmaterials, die bei dessen Anwendung als Implantat äußerst vorteilhaft ist. So kann das Verbundmaterial gut an die Form des zu behandelnden Gewebedefekts angepasst werden, welche häufig unregelmäßig oder zumindest gekrümmt ist, wie beispielsweise bei Schäden des Gelenkknorpels.

Ein weiterer Vorteil des erfindungsgemäßen Verbundmaterials besteht darin, dass die zweite Lage keine wesentliche Volumenverminderung in hydratisiertem Zustand zeigt. Insbesondere bei der Behandlung von Knorpeldefekten, bei der passgenaue Stücke des Verbundmaterials in den umgebenden Knorpel eingesetzt werden, führt ein solches Schrumpfen, wie es bei porösen Materialien auf Basis von Kollagen beobachtet wird, zu erheblichen Problemen. Bevorzugt weist die zweite Lage nach drei Tagen in hydratisiertem Zustand eine Volumenverminderung von weniger als 5% gegenüber dem nach 5 Minuten gemessenen Volumen auf. Am vorteilhaftesten ist es, wenn sich das Volumen der zweiten Lage in hydratisiertem Zustand leicht vergrößert.

Wie bereits angesprochen, bietet das erfindungsgemäße Verbundmaterial den besonderen Vorteil, dass die Resorptionsgeschwindigkeit der zweiten Lage an die jeweiligen Erfordernisse angepasst werden kann. Dies kann insbesondere durch die Wahl der Dichte der zweiten Lage und des Vernetzungsgrades des Gelatine enthaltenden zweiten Materials erfolgen, wobei sowohl eine höhere Dichte als auch ein höherer Vernetzungsgrad tendenziell zu einer Verlängerung der Lebensdauer führen. Im Idealfall erfolgt der Abbau des Materials in dem Maße, in dem die extrazelluläre Matrix von den Zellen synthetisiert wird. Dies kann je nach Zelltyp sehr unterschiedlich sein, wobei insbesondere Knorpelzellen ein vergleichsweise langsames Wachstum aufweisen und daher tendenziell lange Abbauzeiten der zweiten Lage erfordern.

Als Maß für die Resorptions- bzw. Abbaugeschwindigkeit der zweiten Lage bei einer Besiedlung mit Zellen kann auch deren Stabilität ohne Zellbesiedlung unter physiologischen Standardbedingungen (PBS-Puffer, pH 7,2, 37 °C) herangezogen werden. Die physiologischen Bedingungen, denen das Verbundmaterial ausgesetzt ist, sind in erster Linie durch Temperatur, pH-Wert und Ionenstärke gekennzeichnet und können durch eine Inkubation des Verbundmaterials unter den genannten Standardbedingungen simuliert werden, um verschiedene Materialien im Hinblick auf ihr zeitabhängiges Abbauverhalten zu testen und zu vergleichen.

Erfindungsgemäß können durch eine Änderung der Herstellungsbedingungen Verbundmaterialien erhalten werden, bei denen die zweite Lage unter physiologischen Standardbedingungen beispielsweise länger als eine Woche, länger als zwei Wochen oder länger als vier Wochen stabil bleibt.

Der Begriff der Stabilität ist dabei so zu verstehen, dass die zweite Lage ihre ursprüngliche Form (makroskopische Geometrie) während der jeweiligen Zeitdauer im Wesentlichen beibehält und erst anschließend in von außen sichtbarem Umfang abgebaut wird.

In dem Fall, dass die zweite Lage eine Schwammstruktur aufweist, erfolgt dieser Abbau nach der jeweiligen Zeitdauer relativ plötzlich, indem die Schwammstruktur innerhalb weniger Tage zerfällt.

Alternativ kann das Abbauverhalten der zweiten Lage auch durch den Gewichtsverlust unter den oben beschriebenen Bedingungen definiert werden. So können erfindungsgemäße Verbundmaterialien erhalten werden, bei denen die zweite Lage nach einer Woche, nach zwei Wochen oder nach vier Wochen noch zu 70 Gew.% oder mehr erhalten ist.

Ein weiterer Vorteil der Struktur der zweiten Lage ist, dass sie während der Resorptionsphase in einen Hydrogel-artigen Zustand überführbar ist. Ein solcher Übergang in eine Hydrogel-artige Struktur unter physiologischen Standardbedingungen ist insbesondere für die Stabilisierung des Phänotyps von chondrogenen Zellen von Vorteil. Diese Eigenschaften unterstützen im Vergleich zu anderen Biomaterialien eine qualitativ hochwertige Geweberekonstruktion. Andererseits erlauben primär gelartige Biomaterialien eine deutlich schlechtere Zellbesiedelung und kaum ein Einwachsen von Zellen (z.B. nach Mikrofrakturierung) in ihre relativ geschlossenen Strukturen.

Die Überführbarkeit der Struktur der zweiten Lage in eine Hydrogel-Struktur ist dabei vom Vernetzungsgrad abhängig. Sie steht nicht im Gegensatz zu der oben angesprochenen Stabilität, da sich diese auf die makroskopische Geometrie der zweiten Lage bezieht, die auch beim Vorliegen der Hydrogel-Struktur zunächst erhalten bleibt.

Die Abbauzeit der ersten Lage des erfindungsgemäßen Verbundmaterials kann von derjenigen der zweiten Lage abweichen und je nach Fall länger oder kürzer gewählt sein. In jedem Fall verfügt aber die erste Lage auf Grund des erfindungsgemäßen ersten Materials über eine ausreichende Lebensdauer, die gewährleistet, dass die erste Lage auch nach einer Kultivierung von Zellen in der zweiten Lage ihre selbsttragende Eigenschaft aufweist und dem Verbundmaterial die für das Implantieren erforderliche mechanische Festigkeit verleiht.

Wird als erste Lage beispielsweise eine verstärkte Gelatinefolie verwendet, so kann deren Abbauzeit über den Vernetzungsgrad der Gelatine, wie bei dem Gelatine enthaltenden Material der zweiten Lage, in einem gewissen Bereich eingestellt werden. Bei Verwendung einer Membran tierischen Ursprungs ist deren Abbauzeit weitgehend vorgegeben und liegt im Regelfall über derjenigen der zweiten Lage.

Die erste und die zweite Lage des erfindungsgemäßen Verbundmaterials sind bevorzugt unmittelbar miteinander verbunden. Dies kann z.B. dadurch erreicht werden, dass die zweite Lage direkt auf einer Oberfläche der ersten Lage, insbesondere auf der rauen Seite einer tierischen Membran, hergestellt wird.

Bei einer anderen Ausführungsform des erfindungsgemäßen Verbundmaterials sind die beiden Lagen mittels eines Klebers miteinander verbunden sein, wobei der Kleber bevorzugt Gelatine umfasst.

Das erfindungsgemäße Verbundmaterial weist vorzugsweise eine Dicke von 2 bis 5 mm auf, wobei eine Dicke von bis zu 3 mm weiter bevorzugt ist. Die Dikke der ersten Lage beträgt dabei vorzugsweise ca. 1 mm oder weniger.

Die genannte Dicke des Verbundmaterials bezieht sich dabei auf die Gesamtdicke der ersten und der zweiten Lage. Das erfindungsgemäße Verbundmaterial kann aber darüber hinaus noch weitere Lagen umfassen.

Bei einer besonderen Ausführungsform ist eine mit der zweiten Lage verbundene dritte Lage vorgesehen, die auf der Basis eines Gelatine enthaltenden Materials hergestellt ist. Eine solche dritte Lage kann beispielsweise im Fall einer Transplantation von *in vitro* vorkultivierten Zellen dazu dienen, die in der zweiten Lage befindlichen Zellen vor mechanischer Beanspruchung oder vor dem Einwachsen von Fremdzellen zu schützen, oder die Verbindung des Verbundmaterials mit dem benachbarten Gewebe beim Implantieren zu verbessern.

Zum Fixieren eines Implantats an seiner vorgesehenen Stelle im Körper, insbesondere am Knochen im Fall der Knorpelzelltransplantation, kann als dritte Lage z.B. eine Gelatinelösung als Kleber auf die zweite Lage aufgetragen werden. Bevorzugt ist das Gelatine enthaltende Material der dritten Lage, insbesondere die Gelatine selbst, vernetzt. Bevorzugte Vernetzungsmittel sind dabei die im Zusammenhang mit dem zweiten Material der zweiten Lage beschriebenen Verbindungen bzw. Enzyme.

Die dritte Lage weist vorteilhafterweise eine Struktur auf, die das Eindringen von Fremdzellen, z.B. Knochenzellen im Fall der Knorpeltransplantation, verhindert oder erschwert. Bevorzugt weist die dritte Lage daher eine im Wesentlichen geschlossene Struktur auf. Damit ist eine Struktur ohne Poren oder Durchbrüche gemeint, insbesondere eine Folie, z.B. eine Gelatinefolie.

Alternativ kann die dritte Lage aber auch eine poröse Struktur aufweisen, deren mittlerer Porendurchmesser kleiner ist als der mittlere Porendurchmesser der Struktur der zweiten Lage. Dabei handelt es sich bevorzugt um eine Schwammstruktur wie im Zusammenhang mit der zweiten Lage beschrieben, wobei die Schwammstruktur der dritten Lage vorzugsweise einen mittleren Porendurchmesser von 300 µm oder weniger, insbesondere von 100 µm oder weniger aufweist. Die dritte Lage weist bevorzugt auch eine höhere Dichte auf als die zweite Lage, vorzugsweise eine Dichte von 50 g/l oder mehr.

Durch eine dritte Lage mit einer geschlossenen oder porösen Struktur kann auch die Verbindung zwischen dem Verbundmaterial und dem benachbartem Gewebe, insbesondere dem Knochen, verbessert werden. Dabei wird der Vernetzungsgrad des Materials der dritten Lage relativ niedrig gewählt, so dass das Material teilweise vergelt und damit als Kleber wirkt.

Die dritte Lage kann für den Einsatz des Verbundmaterials bei der Transplantation vorkultivierter Zellen, wie z.B. Knorpelzellen oder mesenchymalen Stammzellen, im Hinblick auf eine gute Verträglichkeit mit dem Knochen optimiert werden. Bevorzugt umfasst die dritte Lage dabei ein oder mehrere Calciumphosphate, Apatite oder Mischungen hiervon.

Die dritte Lage des Verbundmaterials wird bevorzugt nach dem Einbringen und ggf. Kultivieren von Zellen in de, zweiten Lage auf diese aufgebracht. Alternativ können die Zellen nach dem Aufbringen der dritten Lage von der Seite her in die zweite Lage eingebracht werden, was insbesondere bei der Herstellung von kleineren Implantaten gut möglich ist.

Der vorliegenden Erfindung liegt ferner die Aufgabe zugrunde, ein Verfahren zur Herstellung des zuvor beschriebenen Verbundmaterials zur Verfügung zu stellen.

Diese Aufgabe wird bei dem eingangs genannten Verfahren erfindungsgemäß dadurch gelöst, dass das Verfahren umfasst:
- Bereitstellen einer ersten selbsttragenden Lage, die ein unter physiologischen Bedingungen unlösliches, resorbierbares, nicht vergelendes erstes Material umfasst;
- Herstellen einer zweiten Lage auf der Basis eines vernetzten, Gelatine enthaltenden zweiten Materials, so dass die zweite Lage eine überwiegend offenporige Struktur aufweist; und
- Verbinden der ersten und der zweiten Lage unter Ausbildung des Verbundmaterials.

Das Verbinden der beiden Lagen kann erfindungsgemäß als abschließender Verfahrensschritt oder im Zuge der Herstellung der zweiten Lage erfolgen.

Im ersteren Fall erfolgt das Verbinden bevorzugt mittels eines Klebers. Dabei umfasst der Kleber vorzugsweise Gelatine, die beispielsweise in Form einer Lösung auf eine oder beide Lagen aufgetragen wird, woraufhin die Lagen zusammengefügt und getrocknet werden.

In dem Fall, dass die erste Lage eine Gelatine enthaltende Matrix umfasst, ist ferner bevorzugt, dass die hergestellte zweite Lage in die erste Lage partiell eingedrückt wird. Dies kann beispielsweise dadurch erfolgen, dass die Gelatine enthaltende Matrix, z.B. eine Gelatinefolie, beim Eindrücken der zweiten Lage in einem plastisch verformbaren Zustand vorliegt, z.B. in einem feuchten Zustand nach deren Herstellung.

Eine bevorzugte Ausführungsform des erfindungsgemäßen Verfahrens betrifft Verbundmaterialien, bei denen die zweite Lage eine Schwammstruktur aufweist. Das Verbinden der beiden Lagen erfolgt dabei im Zuge der Herstellung der zweiten Lage, wobei das Verfahren folgende Schritte umfasst:
a) Bereitstellen der ersten Lage;
b) Herstellen einer wässrigen Lösung des Gelatine enthaltenden zweiten Materials;
c) partielles Vernetzen des gelösten zweiten Materials;
d) Aufschäumen der Lösung;
e) Aufbringen der aufgeschäumten Lösung auf die erste Lage; und
f) Trocknenlassen der aufgeschäumten Lösung unter Ausbildung der zweiten Lage mit einer überwiegend offenporigen Zellstruktur.

Als Ausgangsmaterial für dieses Verfahren kann grundsätzlich Gelatine verschiedener Herkunft und Qualität eingesetzt werden; im Hinblick auf die medizinische Anwendung des Verbundmaterials ist jedoch endotoxinarme Gelatine, wie oben beschrieben, bevorzugt. Die Lösung in Schritt b) weist vorzugsweise eine Gelatinekonzentration von 5 bis 25 Gew.%, insbesondere 10 bis 20 Gew.%, auf.

Neben Gelatine kann das zweite Material bei dem erfindungsgemäßen Verfahren noch weitere Komponenten, beispielsweise andere Biopolymere, enthalten.

Bei der Vernetzungsreaktion in Schritt c) können in diesem Fall eine, mehrere oder alle Komponenten des gelösten zweiten Materials partiell vernetzt werden. Bevorzugt wird dabei insbesondere die Gelatine vernetzt. Die Vernetzung kann chemisch oder enzymatisch erfolgen, wobei bevorzugte Vernetzungsmittel bereits im Zusammenhang mit dem erfindungsgemäßen Verbundmaterial beschrieben wurden.

Eine weiter bevorzugte Ausführungsform dieses Verfahrens umfasst einen weiteren Schritt g), in dem das in der zweiten Lage enthaltene zweite Material zusätzlich vernetzt wird.

Der Vorteil einer solchen zweistufigen Vernetzung besteht darin, dass ein hoher Vernetzungsgrad des zweiten Materials und damit einhergehend die vorteilhaften langen Resorptionszeiten der zweiten Lage erzielt werden können. Dies kann mit einem einstufigen Verfahren unter Erhöhung der Konzentration an Vernetzungsmittel nicht in gleichem Maße verwirklicht werden, da bei einem zu starken Vernetzen des gelösten Materials dieses nicht mehr aufgeschäumt und in Form gebracht werden kann.

Andererseits ist auch ein Vernetzen des Materials, insbesondere der Gelatine, ausschließlich nach der Herstellung des Verbundmaterials nicht geeignet, da hierbei das Material an der von außen zugänglichen Grenzfläche stärker vernetzt als in den inneren Bereichen, was sich in einem inhomogenen Abbauverhalten widerspiegelt.

Die zweite Vernetzung (Schritt g)) kann durch Einwirken einer wässrigen Lösung eines Vernetzungsmittels durchgeführt werden, wobei die oben beschriebenen chemischen oder enzymatischen Vernetzungsmittel zum Einsatz kommen können. Bevorzugt ist jedoch das Einwirken eines gasförmigen Vernetzungsmittels, insbesondere Formaldehyd, welches gleichzeitig einen sterilisierenden Effekt aufweist. Dabei kann das Einwirken des Formaldehyds auf das Verbundmaterial von einer Wasserdampfatmosphäre unterstützt erfolgen.

Bevorzugt wird das Vernetzungsmittel in Schritt c) in einer Menge von 600 bis 5.000 ppm, vorzugsweise 1.000 bis 2.000 ppm, bezogen auf die Gelatine, in die Lösung zugegeben.

Durch eine Variation der Konzentration des Vernetzungsmittels in der Lösung, aber auch durch unterschiedlich hohe Vernetzungsgrade im zweiten Vernetzungsschritt, kann die Lebensdauer der zweiten Lage des Verbundmaterials auf einfache Weise eingestellt werden. So können überraschenderweise Schwammstrukturen erhalten werden, die unter physiologischen Bedingungen z.B. länger als eine Woche, länger als zwei Wochen oder länger als vier Wochen stabil bleiben, wie dies bereits im Zusammenhang mit dem erfindungsgemäßen Verbundmaterial ausführlich erläutert wurde.

Das Aufschäumen (Schritt d)) erfolgt bevorzugt durch Einbringen eines Glases, insbesondere Luft, in die Lösung. Die Dichte und der mittlere Porendurchmesser der herzustellenden Schwammstruktur können dabei in einem weiten Bereich, bevorzugt durch die Intensität des Aufschäumens, eingestellt werden. Neben einer Anpassung des mittleren Porendurchmessers an die Zellen, mit denen die zweite Lage besiedelt werden soll, können über diese Parameter auch die Flexibilität bzw. elastische Verformbarkeit der zweiten Lage (und damit des Verbundmaterials insgesamt) beeinflusst werden. Eine hohe Flexibilität ist z.B. wünschenswert, um ein Implantat an die Form des zu behandelnden Gewebedefekts optimal anpassen zu können.

Die Eigenschaften des nach diesem Verfahren hergestellten Verbundmaterials können in Bezug auf die Stabilität der zweiten Lage noch weiter verbessert werden, wenn das Verbundmaterial nach dem zweiten Vernetzungsschritt einer thermischen Nachbehandlung bei vermindertem Druck ausgesetzt wird. Diese Nachbehandlung wird bevorzugt bei Temperaturen von 80 bis 160 °C durchgeführt, da unterhalb von 80 °C die beobachteten Effekte relativ schwach ausgeprägt sind und oberhalb von 160 °C eine unerwünschte Verfärbung der Gelatine auftreten kann. Am meisten bevorzugt sind Werte im Bereich von 90 bis 120 °C.

Unter vermindertem Druck sind dabei Drücke unterhalb des Atmosphärendrucks zu verstehen, wobei möglichst geringe Druckwerte, im Idealfall ein Vakuum, bevorzugt sind.

Die thermische Nachbehandlung wirkt sich in zweierlei Hinsicht vorteilhaft aus. Zum einen erfolgt unter den oben genannten Temperatur- und Druckbedingungen eine weitere, dehydrothermale Vernetzung der Gelatine, indem verschiedene Aminosäureseitenketten unter Wasserabspaltung miteinander reagieren. Dies wird dadurch begünstigt, dass das abgespaltene Wasser durch den geringen Druck aus dem Gleichgewicht entfernt wird. Durch die thermische Nachbehandlung kann somit ein höherer Vernetzungsgrad bei gleicher Menge an Vernetzungsmitteln erzielt werden bzw. die Menge an Vernetzungsmitteln kann bei vergleichbarem Vernetzungsgrad reduziert werden.

Der weitere Vorteil der thermischen Nachbehandlung besteht darin, dass der in der zweiten Lage verbleibende Restgehalt an nicht verbrauchtem Vernetzungsmittel deutlich reduziert werden kann.

Um eine gute Bioverträglichkeit des Verbundmaterials sicherzustellen, wird bei dem erfindungsgemäßen Verfahren vorzugsweise überschüssiges, nicht reagiertes Vernetzungsmittel aus der zweiten Lage entfernt. Dies kann z.B. durch mehrtägiges Entgasen des Verbundmaterials unter Normaldruck und/oder durch Waschen mit einem flüssigen Medium erfolgen, wobei letzteres je nach Konzentration des Vernetzungsmittels, Größe des Verbundmaterials usw. ebenfalls einen Zeitraum von einem Tag bis zu einer Woche erfordert.

Da durch die oben beschriebene thermische Nachbehandlung einerseits die Menge an eingesetztem Vernetzungsmittel reduziert werden kann und darüber hinaus überschüssiges Vernetzungsmittel durch die erhöhte Temperatur und den verminderten Druck aus dem Verbundmaterial entfernt wird, kann durch diesen zusätzlichen Verfahrensschritt eine deutliche Verringerung des Restgehaltes an Vernetzungsmittel bereits innerhalb von ca. 4 bis 10 Stunden erreicht werden.

Bei einer besonderen Ausführungsform des erfindungsgemäßen Verfahrens umfasst dieses ferner das Aufbringen einer dritten Lage auf die zweite Lage des Verbundmaterials. Dies kann sowohl vor als auch nach dem Einbringen von Zellen in die zweite Lage erfolgen. Vorteile und Ausführungsformen einer dritten Lage wurden bereits im Zusammenhang mit dem erfindungsgemäßen Verbundmaterial beschrieben.

Die Erfindung betrifft des Weiteren die Verwendung des beschriebenen Verbundmaterials für den Einsatz im human- und veterinärmedizinischen Bereich, insbesondere für die Herstellung von Implantaten.

Das erfindungsgemäße Verbundmaterial ist hervorragend für die Besiedlung mit oder für das Einwachsen von menschlichen oder tierischen Zellen, geeignet. Für die Transplantation *in vitro* isolierter und/oder vorkultivierter Zellen wird das Verbundmaterial z.B. mit Chondrozyten, mesenchymalen Stammzellen, Periostzellen oder Fibroblasten, besiedelt, die in einem geeigneten Nährmedium auf die zweite Lage ausgesät und bevorzugt in deren überwiegend offenporige Struktur eingelagert werden. Aufgrund der hohen Stabilität des Materials können die Zellen *in vitro* mehrere Wochen wachsen und proliferieren.

Die Erfindung betrifft weiterhin Implantate, insbesondere Gewebeimplantate, die das erfindungsgemäße Verbundmaterial und menschliche oder tierische Zellen umfassen.

Bei einer Ausführungsform des erfindungsgemäßen Implantats umfasst dieses lediglich einwachsende Zellen, die sich in der zweiten Lage einlagern. In diesem Fall erfolgt keine Beladung von Zellen *in vitro,* sondern das Verbundmaterial wird, z.B. nach vorausgegangener Mikrofrakturierung, unmittelbar implantiert. Die im Blutkoagel befindlichen Zellen besiedeln das Biomaterial dann *in vivo*.

Bei einer weiteren Ausführungsform des erfindungsgemäßen Implantats sind die Zellen in der zweiten Lage kultiviert, d.h. vor der Implantation wird eine Besiedelung und Kultivierung *in vitro*, wie oben beschrieben, durchgeführt.

Die *in vivo* einwachsenden und/oder die *in vitro* eingesäten Zellen sind vorzugsweise im Wesentlichen gleichmäßig in der zweiten Lage des Verbundmaterials verteilt. Dadurch wird die Ausbildung einer dreidimensionalen Gewebestruktur ermöglicht.

Die erfindungsgemäßen Implantate werden für die Behandlung von Gewebedefekten verwendet, wie dies bereits mehrfach angesprochen wurde. Bevorzugte Anwendungen betreffen die Behandlung von Schäden und/oder Verletzungen des menschlichen oder tierischen Knorpelgewebes, insbesondere im Rahmen einer autologen Knorpelzelltransplantation oder die matrixgekoppelte Mikrofrakturierung, die Behandlung von Defekten der menschlichen Rotatorenmanschette der Schulter, knöcherne Defekte (z.B. Sinusaugmentation des Kiefers) sowie die Behandlung von Schäden, Verletzungen und/oder Verbrennungen der menschlichen oder tierischen Haut.

Auch hier ermöglicht das erfindungsgemäße Verbundmaterial aufgrund seiner Struktur eine geschützte und gerichtete Defektsanierung im Sinne einer Guided Tissue Regeneration.

Die Erfindung betrifft, wie bereits erwähnt, schließlich auch ein Verfahren zur zellbasierten Knorpelregeneration mit *in vitro* kultivierten Zellen. Das Verfahren umfasst die Entnahme von Chondrozyten oder von Stammzellen autologer oder allogener Herkunft, das Aussäen der potentiell chondrogenen Zellen auf die zweite Lage eines erfindungsgemäßen Verbundmaterials, sowie das Einsetzen des Verbundmaterials mit den Zellen an die Stelle des Knorpeldefekts eines Patienten.

Die Form des Verbundmaterials ist dabei bevorzugt an die Form des Knorpeldefekts angepasst. Weiter ist bevorzugt, dass die erste Lage des Verbundmaterials beim Einsetzen in den Knorpel nach außen orientiert ist.

Bei einer bevorzugten Ausführungsform des Verfahrens werden die ausgesäten Zellen vor dem Implantieren des Verbundmaterials *in vitro* kultiviert; vorzugsweise für einen Zeitraum von 4 bis 14 Tagen.

Diese und weitere Vorteile der Erfindung werden anhand der folgenden Beispiele unter Bezugnahme auf die Figuren näher erläutert. Es zeigen im Einzelnen:
- Figur 1:: Lichtmikroskopische Aufnahme eines Querschnitts durch ein erfindungsgemäßes Verbundmaterial;
- Figur 2:: Lichtmikroskopische Aufnahme der zweiten Lage eines erfindungsgemäßen Verbundmaterials nach zweiwöchiger Besiedlung mit Chondrozyten; und
- Figur 3:: fotografische Darstellung eines erfindungsgemäßen Verbundmaterials nach vierwöchiger Besiedlung mit Chondrozyten.

### Beispiel 1: Herstellung und Eigenschaften eines erfindungsgemäßen Verbundmaterials

Dieses Beispiel betrifft die Herstellung eines erfindungsgemäßen Verbundmaterials, bei dem als erste Lage eine Perikardmembran vom Rind eingesetzt wurde.

Um eine möglichst hohe Bioverträglichkeit zu gewährleisten, wurde eine Perikardmembran verwendet, die weitestgehend von Fetten, Enzymen und anderen Proteinen befreit worden war. Durch Lyophilisieren der Membran wurde dabei eine lockere Faserstruktur des Kollagens erhalten. Derartige Perikardmembranen, die im Wesentlichen aus Kollagen-Typ-I bestehen, werden auch als Ersatz von Bindegewebestrukturen in der Neurochirurgie eingesetzt.

Drei Stücke dieser Perikardmembran mit einer Größe von jeweils ca. 10 x 10 cm wurden mit der rauen Seite nach oben auf ca. 3 cm hohen Unterlageblöcken fixiert. Diese drei Blöcke wurden dann auf dem Boden einer Kastenform mit einer Länge und Breite von 40 x 20 cm und einer Höhe von 6 cm verteilt.

Zur Herstellung der zweiten Lage des Verbundmaterials wurde zunächst eine 12 Gew.-%ige Lösung von Schweineschwartengelatine mit einer Bloomstärke von 300 g hergestellt, indem die Gelatine bei 60 °C in Wasser gelöst wurde. Die Lösung wurde mittels Ultraschall entgast und mit der entsprechenden Menge einer wässrigen Formaldehydlösung (1,0 Gew.%ig, Raumtemperatur) versetzt, so dass 2.000 ppm Formaldehyd, bezogen auf die Gelatine, vorlagen.

Die homogenisierte Mischung wurde auf 45 °C temperiert und nach einer Reaktionszeit von 5 Minuten ca. 30 Minuten lang maschinell mit Luft aufgeschäumt, wobei ein Gelatineschaum mit einer Nassdichte von 130 g/l erhalten wurde.

Die Kastenform mit den aufgespannten Perikardmembranen wurde mit dieser aufgeschäumten Gelatinelösung, die eine Temperatur von ca. 27 °C aufwies, aufgefüllt, und der Gelatineschaum für ca. 6 bis 8 Tage bei einer Temperatur von 26 °C und einer relativen Luftfeuchtigkeit von 10% getrocknet.

Nach dem Trocknen bildete der Gelatineschaum ein schnittfestes Material mit einer überwiegend offenporigen Schwammstruktur (im Folgenden als Gelatineschwamm bezeichnet). Durch das Trocknen des Gelatineschaums in unmittelbarem Kontakt mit der Perikardmembran ergab sich in den überwiegenden Bereichen eine stabile Verbindung zwischen den beiden Materialien, was durch die Rauheit der verwendeten Oberfläche der Perikardmembran zusätzlich begünstigt wurde.

Es wurden ca. 1,5 x 1,5 cm große Stücke der Perikardmembran mit dem daran anhaftenden Gelatineschwamm ausgeschnitten, wobei von dem Gelatineschwamm oberhalb der Membran soviel weggeschnitten wurde, dass die Stücke eine Dicke von ca. 3 mm aufwiesen.

Der die zweite Lage des Verbundmaterials bildende Gelatineschwamm wies im vorliegenden Beispiel nach dem Trocknen eine Dichte von 22 g/l und einen mittleren Porendurchmesser von etwa 250 µm auf. Durch eine Änderung der Herstellungsbedingungen können diese Parameter in einem weiten Bereich beeinflusst werden, um so den mittlere Porendurchmesser an die Größe der Zellen anzupassen, mit denen das Verbundmaterial besiedelt werden soll.

So konnten durch eine Änderung der Intensität des Aufschäumens beispielsweise auch Verbundmaterialien nach der oben beschriebenen Vorgehensweise hergestellt werden, bei denen der Gelatineschwamm eine Nassdichte von 175 g/l, einer Trockendichte von 27 g/l und einem mittleren Porendurchmesser von etwa 200 µm, bzw. eine Nassdichte von 300 g/l, eine Trockendichte von 50 g/l und einem mittleren Porendurchmesser von etwa 125 µm aufwies.

Um eine ausreichend lange Lebensdauer der zweiten Lage des Verbundmaterials zu gewährleisten, wurde die Gelatine einem zweiten Vernetzungsschritt unterzogen. Hierfür wurden jeweils 1,5 x 1,5 cm großen Stücke des Trägermaterials in einem Exsikkator für 17 Stunden dem Gleichgewichtsdampfdruck einer 17 Gew.%igen wässrigen Formaldehydlösung bei Raumtemperatur ausgesetzt, wobei der Exsikkator zuvor zwei- bis dreimal evakuiert und wieder belüftet wurde.

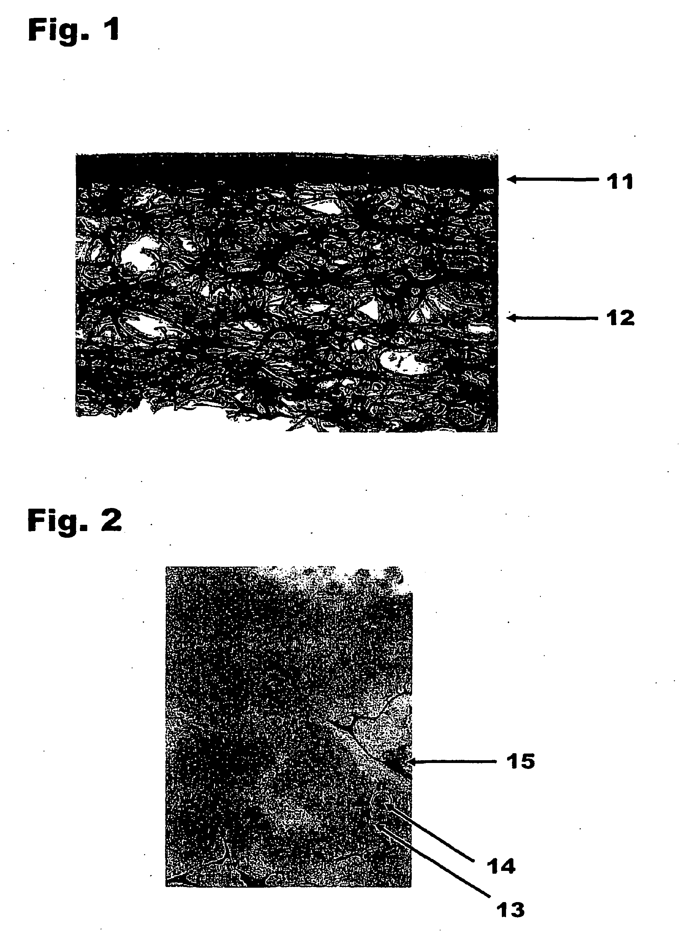

In der Figur 1 ist eine lichtmikroskopische Aufnahme eines Querschnitts durch das auf diese Weise hergestellte erfindungsgemäße Verbundmaterial dargestellt. Dabei wird die erste Lage von der Perikardmembran 11 und die zweite Lage von dem Gelatineschwamm 12 mit dem mittleren Porendurchmesser von etwa 250 µm gebildet. Die überwiegend offenporige Struktur der zweiten Lage ist deutlich zu erkennen.

Um den Einfluss des zweiten Vernetzungsschrittes zu demonstrieren, wurde das Auflösungsverhalten des zweifach bzw. einfach vernetztem Verbundmaterials miteinander verglichen. Hierfür wurden ca. 1,5 x 1,5 cm große Probenstücke des oben beschriebenen Verbundmaterials sowie gleichgroße Referenzproben, die keiner Nachvernetzung in der Gasphase unterzogen worden waren, in jeweils 75 ml PBS-Puffer (pH 7,2) gelegt und bei 37 °C gelagert. Dabei zeigte sich, dass bei den Proben des Verbundmaterials mit einfach vernetzter Gelatine die zweite Lage bereits nach drei Tagen vollständig aufgelöst war. Demgegenüber war die zweite Lage bei den Proben, die der oben beschriebenen Nachvernetzung in der Gasphase unterzogen worden waren, selbst nach 14 Tagen noch zu über 80 Gew. % erhalten. An der Perikardmembran der ersten Lage war bei allen Proben nach 14 Tagen noch kein Abbau zu erkennen.

Es muss in diesem Zusammenhang natürlich berücksichtigt werden, dass bei einer Besiedlung des Verbundmaterials mit Zellen bzw. im Körper die tatsächlichen Abbauzeiten sich von den in diesem Versuch gefundenen Zeiten unterscheiden können. Dennoch zeigt dieses Ergebnis, dass durch die zweistufige Vernetzung der Gelatine die Lebensdauer der zweiten Lage unter physiologischen Bedingungen deutlich verlängert werden kann, was für die medizinische Anwendung des Verbundmaterials, insbesondere im Bereich der Knorpelzelltransplantation, von erheblicher Bedeutung ist.

Darüber hinaus ist es möglich, die Lebensdauer durch eine Variation der Herstellungsbedingungen gezielt zu beeinflussen. Insbesondere führen ein höherer Anteil an Vernetzungsmittel in der Gelatinelösung, eine höhere Dichte des Gelatineschwamms und/oder eine längere Einwirkzeit des Vernetzungsmittels in der Gasphase zu einer Verlängerung der Abbauzeiten.

Zusätzlich kann die Lebensdauer auch durch eine thermische Nachbehandlung weiter verlängert werden. Dies kann im vorliegenden Beispiel dadurch geschehen, dass die Probenstücke nach dem zweiten Vernetzungsschritt vakuum-entgast und dann mit Hilfe eines Rotationsverdampfers für sechs Stunden bei 105 °C unter einem Vakuum von ca. 14 mbar gehalten werden.

Wird eine derartige thermische Nachbehandlung durchgeführt, so kann die Einwirkzeit des Formaldehyds im zweiten Vernetzungsschritt von 17 auf beispielsweise zwei oder fünf Stunden verkürzt werden, um zu Verbundmaterialien mit einer Lebensdauer der zweiten Lage im Bereich von einer bis vier Wochen zu gelangen. Durch diese Vorgehensweise weist zudem die zweite Lage einen um bis zu 40% geringeren Restgehalt an überschüssigem Formaldehyd auf. Dadurch verkürzt sich die Zeit, während der die erfindungsgemäßen Verbundmaterialien vor ihrer Implantation oder ihrer Besiedelung mit Zellen gewaschen werden müssen.

### Beispiel 2: Herstellung eines weiteren erfindungsgemäßen Verbundmaterials

Dieses Beispiel betrifft die Herstellung eines erfindungsgemäßen Verbundmaterials, bei dem als erste Lage eine mit Baumwollfasern verstärkte Gelatinefolie eingesetzt wurde.

Zur Herstellung der ersten Lage wurden 20 g Schweineschwartengelatine (Bloom-Stärke 300 g) in einer Mischung aus 71 g Wasser und 9 g Glycerin bei 60 °C gelöst und die Lösung mittels Ultraschall entgast. Das Glycerin diente dabei als Weichmacher, um eine gewisse Flexibilität und Dehnbarkeit der fertigen Gelatinefolie zu gewährleisten.

Als Verstärkungsstoff wurden 1 g kurze Baumwollfasern (Linters) in 25 g Wasser aufgeschlämmt und diese Suspension unter ständigem Rühren zu der Lösung von Gelatine und Glycerin gegeben. Nach Zugabe von 2 g einer wässrigen Formaldehydlösung (2,0 Gew.%ig, Raumtemperatur) zu der Lösung wurde diese homogenisiert und bei ca. 60 °C in einer Dicke von 1 mm auf eine Polyethylenunterlage gerakelt.

Nach Trocknen bei 25 °C und einer relativen Luftfeuchtigkeit von 30% während etwa zwei Tagen wurde die hergestellte Folie von der PE-Unterlage abgezogen.

Die faserverstärkte Gelatinefolie wies eine Dicke von etwa 200 bis 250 µm und eine Reißfestigkeit von ca. 22. N/mm² bei einer Reißdehnung von ca. 45% auf. Eine entsprechend hergestellte, unverstärkte Gelatinefolie wies demgegenüber eine Reißfestigkeit von ca. 15 N/mm² auf.

Die Herstellung der zweiten Lage erfolgte wie in Beispiel 1 beschrieben, wobei die Kastenform (ohne Perikardmembranen) mit der aufgeschäumten Gelatinelösung gefüllt wurde. Von dem getrockneten Gelatineschwamm wurde eine ca. 2 bis 3 mm dicke Schicht geschnitten.

Die faserverstärkte Gelatinefolie (erste Lage) und der Gelatineschwamm (zweite Lage) wurden mittels einer Lösung aus Knochengelatine (Bloom-Stärke 160 g) vollflächig miteinander verklebt und das hergestellte Verbundmaterial anschließend einer zweiten Vernetzung mit Formaldehyd in der Gasphase, wie in Beispiel 1 beschrieben, unterzogen.

Statt der Verwendung einer Gelatinelösung als Kleber kann die Verbindung zwischen den beiden Lagen auch so hergestellt werden, dass der bereits getrocknete Schwamm in die gerakelte, noch nicht getrocknete Folie partiell eingedrückt wird. Auch auf diese Weise kann eine stabile, vollflächige Verbindung erzielt werden.

Bei einer Abwandlung dieses Beispiels wurden die Baumwollfasern durch Kollagenfasern ersetzt. Die Herstellung der Folien erfolgte wie oben beschrieben, mit der Ausnahme, dass zu der Lösung von Gelatine und Glycerin eine Suspension von 5 g Kollagenfasern in 60 g Wasser bzw. 10 g Kollagenfasern in 90 g Wasser gegeben wurde.

Die getrockneten Folien wiesen eine Reißfestigkeit von ca. 25 N/mm² bei einer Reißdehnung von ca. 40% (5 g Fasern) bzw. eine Reißfestigkeit von ca. 30 N/mm² bei einer Reißdehnung von ca. 27% (10 g Fasern) auf, während die Reißfestigkeit einer entsprechenden unverstärkten Folie bei ca. 17 N/mm² lag.

Durch den zweiten Vernetzungsschritt in der Gasphase erhöhten sich die Reißfestigkeiten der mit Kollagenfasern verstärkten Folien nochmals auf ca. 28 N/mm² (5 g Fasern) bzw. auf ca. 33 N/mm² (10 g Fasern).

### Beispiel 3: Besiedlung eines erfindungsgemäßen Verbundmaterials mit Chondrozyten

Dieses Beispiel beschreibt die Besiedlung des gemäß Beispiel 1 hergestellten, zweistufig vernetzten Verbundmaterials mit Chondrozyten (Knorpelzellen) vom Schwein. Dies kann als Modellversuch für eine Transplantation von chondrogenen Zellen angesehen werden, bei der humane Zellen, wie z.B. artikuläre Chondrozyten, *in vitro* auf dem Trägermaterial kultiviert werden.

Als Kulturmedium wurde DMEM/10%FCS/Glutamin/Pen/Strep verwendet, welches ein Standardmedium für die Kultivierung von Säugetierzellen ist. Vor der Besiedlung des Verbundmaterials wurde dieses mit Kulturmedium gewaschen Auf die zweite Lage des Verbundmaterials wurden anschließend pro cm² eine Million Chondrozyten, suspendiert in 150 µl Kulturmedium, ausgesät. Das Trägermaterial wurde anschließend vier Wochen bei 37 °C in Kulturmedium inkubiert.

Die Figur 2 zeigt eine Lichtmikroskopische Aufnahme der zweiten Lage des Verbundmaterials nach zweiwöchiger Inkubation. Die Zellkerne 13 der Chondrozyten sind sehr regelmäßig über das gesamte Volumen verteilt. Die Schwammstruktur der zweiten Lage wurde im Verlauf der zwei Wochen zu einem großen Teil abgebaut und durch die von den Chondrozyten synthetisierte extrazelluläre Matrix 14 ersetzt. Reste der Schwammstruktur 15 sind beispielsweise noch am rechten Rand der Abbildung zu erkennen.

An dieser Stelle sei nochmals darauf hingewiesen, dass der Abbau des Materials der zweiten Lage unter diesen Bedingungen schneller erfolgt als bei dem in Beispiel 1 beschriebenen Versuch in PBS-Puffer, was u.a. auf einen enzymatischen Abbau der Gelatine zurückzuführen ist.

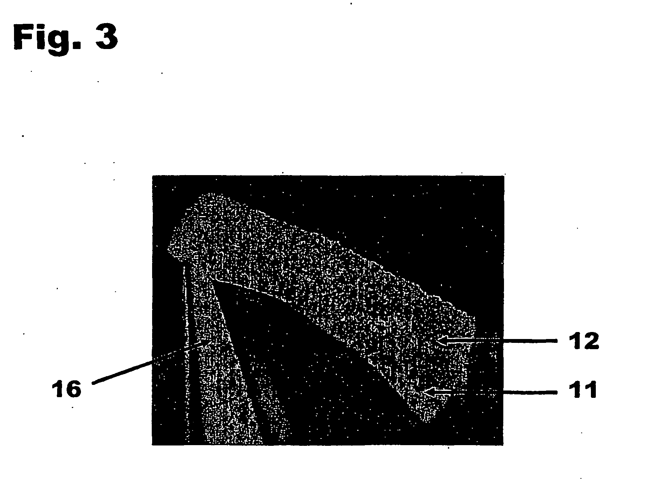

Die Figur 3 zeigt eine fotografische Darstellung des erfindungsgemäßen Verbundmaterials nach einer Besiedlungszeit von vier Wochen. Das Verbundmaterial wird mit einer Pinzette 16 gehalten, wobei die zweite Lage nach oben orientiert ist. Aufgrund der äußerst festen Perikardmembran 11 weist das Verbundmaterial nach wie vor eine hohe Formstabilität auf und ist daher gut handhabbar. Zudem besteht auch nach vier Wochen eine stabile Verbindung zwischen der Perikardmembran 11 und dem Gelatineschwamm 12 bzw. der darin gebildeten extrazellulären Matrix.

Die Ergebnisse dieses Versuches zeigen, dass entsprechende Gewebeimplantate, die unter Verwendung von humanen chondrogenen Zellen hergestellt werden können, für den Einsatz im Rahmen einer zellbasierten Knorpelregeneration in hohem Maße geeignet sind.

## Patentansprüche

1. Verbundmaterial, umfassend
- eine erste selbsttragende Lage, die ein unter physiologischen Bedingungen unlösliches, resorbierbares, nicht vergelendes erstes Material umfasst; und
- eine zweite Lage, hergestellt auf der Basis eines vernetzten, Gelatine enthaltenden zweiten Materials, wobei die zweite Lage eine überwiegend offenporige Struktur aufweist.

2. Verbundmaterial nach Anspruch 1, wobei das unlösliche, resorbierbare, nicht vergelende erste Material ein Flächenmaterial auf Basis von Kollagen ist.

3. Verbundmaterial nach Anspruch 2, wobei das Flächenmaterial auf Basis von Kollagen eine natürliche Membran tierischen Ursprungs ist.

4. Verbundmaterial nach Anspruch 3, wobei die tierische Membran eine Perikardmembran ist.

5. Verbundmaterial nach einem der Ansprüche 2 bis 4, wobei die tierische Membran eine raue Seite aufweist, welche zu der zweiten Lage hin orientiert ist.

6. Verbundmaterial nach Anspruch 1, wobei das erste Material einen Verstärkungsstoff umfasst.

7. Verbundmaterial nach Anspruch 6, wobei der Verstärkungsstoff ausgewählt ist aus partikulären und/oder molekularen Verstärkungsstoffen.

8. Verbundmaterial nach Anspruch 6 oder 7, wobei die erste Lage eine Matrix umfasst, in welche der Verstärkungsstoff des ersten Materials eingebettet ist.

9. Verbundmaterial nach Anspruch 8, wobei die Matrix auf der Basis eines vernetzten, Gelatine enthaltenden Materials hergestellt ist.

10. Verbundmaterial nach einem der Ansprüche 1 bis 9, wobei die zweite Lage eine Faserstruktur aufweist.

11. Verbundmaterial nach Anspruch 10, wobei die Faserstruktur ein Gewebe, ein Gewirk oder ein Vlies umfasst.

12. Verbundmaterial nach einem der Ansprüche 1 bis 9, wobei die zweite Lage eine Schwammstruktur aufweist.

13. Verbundmaterial nach einem der Ansprüche 1 bis 12, ferner umfassend eine mit der zweiten Lage verbundene dritte Lage.

14. Verbundmaterial nach Anspruch 13, wobei die dritte Lage auf der Basis eines Gelatine enthaltenden Materials hergestellt ist.

15. Verbundmaterial nach Anspruch 13 oder 14, wobei die dritte Lage eine geschlossene Struktur aufweist.

16. Verbundmaterial nach Anspruch 13 oder 14, wobei die dritte Lage eine poröse Struktur aufweist, deren mittlerer Porendurchmesser kleiner ist als der mittlere Porendurchmesser der Struktur der zweiten Lage.

17. Verfahren zur Herstellung eines Verbundmaterials nach einem der Ansprüche 1 bis 16, umfassend
- Bereitstellen einer ersten selbsttragenden Lage, die ein unter physiologischen Bedingungen unlösliches, resorbierbares, nicht vergelendes erstes Material umfasst;
- Herstellen einer zweiten Lage auf der Basis eines vernetzten, Gelatine enthaltenden zweiten Materials, so dass die zweite Lage eine überwiegend offenporige Struktur aufweist; und
- Verbinden der ersten und der zweiten Lage unter Ausbildung des Verbundmaterials.

18. Verfahren nach Anspruch 17, umfassend die Schritte:
a) Bereitstellen der ersten Lage;
b) Herstellen einer wässrigen Lösung des Gelatine enthaltenden zweiten Materials;
c) partielles Vernetzen des gelösten zweiten Materials;
d) Aufschäumen der Lösung;
e) Aufbringen der aufgeschäumten Lösung auf die erste Lage; und
f) Trocknenlassen der aufgeschäumten Lösung unter Ausbildung der zweiten Lage mit einer überwiegend offenporigen Struktur.

19. Verfahren nach Anspruch 18, ferner umfassend den Schritt:
g) Vernetzen des in der zweiten Lagen enthaltenen zweiten Materials.

20. Verfahren nach Anspruch 18 oder 19, wobei die Vernetzung in Schritt g) durch das Einwirken eines Vernetzungsmittels in der Gasphase durchgeführt wird.

21. Verfahren nach einem der Ansprüche 18 bis 20, wobei das Verbundmaterial einer thermischen Nachbehandlung bei reduziertem Druck unterworfen wird.

22. Verfahren nach einem der Ansprüche 17 bis 21, ferner umfassend das Aufbringen einer dritten Lage auf die zweite Lage des Verbundmaterials.

23. Verwendung eines Verbundmaterials nach einem der Ansprüche 1 bis 16 zur Kultivierung von Zellen *in vitro.*

24. Verwendung eines Verbundmaterials nach einem der Ansprüche 1 bis 16 zur Herstellung von Implantaten.

25. Implantat, umfassend ein Verbundmaterial nach einem der Ansprüche 1 bis 16 sowie Zellen, die in der zweiten Lage eingelagert sind.

26. Implantat, umfassend ein Verbundmaterial nach einem der Ansprüche 1 bis 16 sowie Zellen, die in der zweiten Lage kultiviert sind.

## Claims

1. Composite material, comprising
- a first self-supporting layer, which comprises a first material which is insoluble, resorbable and non-gelling under physiological conditions; and
- a second layer, produced on the basis of a cross-linked second material containing gelatin, the second layer having a mainly open-pored structure.

2. Composite material according to Claim 1, the insoluble, resorbable and non-gelling first material being a sheet material on the basis of collagen.

3. Composite material according to Claim 2, the sheet material on the basis of collagen being a natural membrane of animal origin.

4. Composite material according to Claim 3, the animal membrane being a pericardial membrane.

5. Composite material according to any of Claims 2 to 4, the animal membrane having a rough side which is oriented toward the second layer.

6. Composite material according to Claim 1, the first material comprising a reinforcing material.

7. Composite material according to Claim 6, the reinforcing material being selected from particulate and/or molecular reinforcing materials.

8. Composite material according to Claim 6 or 7, the first layer comprising a matrix in which the reinforcing material of the first material is embedded.

9. Composite material according to Claim 8, the matrix being produced on the basis of a cross-linked material containing gelatin.

10. Composite material according to any of Claims 1 to 9, the second layer having a fibre structure.

11. Composite material according to Claim 10, the fibre structure being a textile, a knitted material, or a non-woven material.

12. Composite material according to any of Claims 1 to 9, the second layer having a sponge structure.

13. Composite material according to any of Claims 1 to 12, further comprising a third layer bonded to the second layer.

14. Composite material according to Claim 13, the third layer being produced on the basis of a material containing gelatin.

15. Composite material according to Claim 13 or 14, the third layer having a closed structure.

16. Composite material according to Claim 13 or 14, the third layer having a porous structure, the average pore diameter of which is less than the average pore diameter of the structure of the second layer.

17. Method for producing a composite material according to any of Claims 1 to 16, comprising
- providing a first self-supporting layer, which comprises a first material which is insoluble, resorbable and non-gelling under physiological conditions;
- producing a second layer on the basis of a cross-linked second material containing gelatin, so that the second layer has a mainly open-pored structure; and
- bonding the first and the second layer, whereby the composite material is formed.

18. Method according to Claim 17, comprising the steps of:
a) providing the first layer;
b) preparing an aqueous solution of the second material containing gelatin;
c) partially cross-linking the dissolved second material;
d) foaming the solution;
e) applying the foamed solution to the first layer; and
f) leaving the foamed solution to dry, whereby the second layer is formed with a mainly open-pored structure.

19. Method according to Claim 18, further comprising the step of:
g) cross-linking the second material comprised in the second layer.

20. Method according to Claim 18 or 19, the cross-linking in step g) being carried out by the action of a cross-linking agent in the gas phase.

21. Method according to any of Claims 18 to 20, the composite material being subjected to a thermal after-treatment at reduced pressure.

22. Method according to any of Claims 17 to 21, further comprising application of a third layer to the second layer of the composite material.

23. Use of a composite material according to any of Claims 1 to 16 for cultivation of cells *in vitro*.

24. Use of a composite material according to any of Claims 1 to 16 for producing implants.

25. Implant, comprising a composite material according to any of Claims 1 to 16 and cells which are embedded in the second layer.

26. Implant, comprising a composite material according to any of Claims 1 to 16 and cells which are cultivated in the second layer.

## Revendications

1. Matériau composite comprenant
- une première couche autoporteuse qui comprend un premier matériau insoluble, résorbable, non gélifiant dans les conditions physiologiques ; et
- une deuxième couche produite à base d'un deuxième matériau réticulé, contenant de la gélatine, où la deuxième couche présente une structure principalement à pores ouverts.

2. Matériau composite selon la revendication 1 où le premier matériau insoluble, résorbable, non gélifiant est un matériau de surface à base de collagène.

3. Matériau composite selon la revendication 2 où le matériau de surface à base de collagène est une membrane naturelle d'origine animale.

4. Matériau composite selon la revendication 3 où la membrane animale est une membrane de péricarde.

5. Matériau composite selon l'une des revendications 2 à 4 où la membrane animale présente un côté rugueux qui est orienté vers la deuxième couche.

6. Matériau composite selon la revendication 1 où le premier matériau comprend une substance de renforcement.

7. Matériau composite selon la revendication 6 où la substance de renforcement est choisie parmi les substances de renforcement particulaires et/ou moléculaires.

8. Matériau composite selon la revendication 6 ou 7 où la première couche comprend une matrice dans laquelle est incluse la substance de renforcement du premier matériau.

9. Matériau composite selon la revendication 8 où la matrice est produite à base d'un matériau réticulé contenant de la gélatine.

10. Matériau composite selon l'une des revendications 1 à 9 où la deuxième couche présente une structure fibreuse.

11. Matériau composite selon la revendication 10 où la structure fibreuse comprend un tissu, un tissu à maille ou un non-tissé.

12. Matériau composite selon l'une des revendications 1 à 9 où la deuxième couche présente une structure d'éponge.

13. Matériau composite selon l'une des revendications 1 à 12 comprenant en outre une troisième couche liée à la deuxième couche.

14. Matériau composite selon la revendication 13 où la troisième couche est produite à base d'un matériau contenant de la gélatine.

15. Matériau composite selon la revendication 13 ou 14 où la troisième couche présente une structure fermée.

16. Matériau composite selon la revendication 13 ou 14 où la troisième couche présente une structure poreuse dont le diamètre poreux moyen est inférieur au diamètre poreux moyen de la structure de la deuxième couche.

17. Procédé pour produire un matériau composite selon l'une des revendications 1 à 16 comprenant
- la fourniture d'une première couche autoporteuse qui comprend un premier matériau insoluble, résorbable, non gélifiant dans les conditions physiologiques ;
- la production d'une deuxième couche à base d'un deuxième matériau réticulé contenant de la gélatine de sorte que la deuxième couche présente une structure principalement à pores ouverts ; et
- la liaison de la première et de la deuxième couche avec formation du matériau composite.

18. Procédé selon la revendication 17 comprenant les étapes :
a) fourniture de la première couche ;
b) production d'une solution aqueuse du deuxième matériau contenant de la gélatine ;
c) réticulation partielle du deuxième matériau dissous ;
d) moussage de la solution ;
e) application de la solution ayant subi le moussage sur la première couche ; et
f) séchage de la solution ayant subi le moussage avec formation de la deuxième couche ayant une structure principalement à pores ouverts.

19. Procédé selon la revendication 18 comprenant en outre l'étape :
g) réticulation du deuxième matériau contenu dans la deuxième couche.

20. Procédé selon la revendication 18 ou 19 où la réticulation dans l'étape g) est réalisée par l'action d'un agent réticulant en phase gazeuse.

21. Procédé selon l'une des revendications 18 à 20 où le matériau composite est soumis à un post-traitement thermique sous pression réduite.

22. Procédé selon l'une des revendications 17 à 21 comprenant en outre l'application d'une troisième couche sur la deuxième couche du matériau composite.

23. Utilisation d'un matériau composite selon l'une des revendications 1 à 16 pour la culture de cellules *in vitro.*

24. Utilisation d'un matériau composite selon l'une des revendications 1 à 16 pour la production d'implants.

25. Implant comprenant un matériau composite selon l'une des revendications 1 à 16 ainsi que des cellules qui sont incorporées dans la deuxième couche.

26. Implant comprenant un matériau composite selon l'une des revendications 1 à 16 ainsi que les cellules qui sont cultivées dans la deuxième couche.
